# EUROPEAN PATENT APPLICATION

(11) **EP 4 702 915 A1**
(43) Date of publication of application: **04.03.2026**
(21) Application number: 24197520.0
(22) Date of filing: 30.08.2024
(51) Int. Cl.: A61B 3/00, A61B 3/117, A61F 9/00, A61B 3/10, A61B 3/107

(54) **METHOD, SYSTEM AND COMPUTER PROGRAM FOR SELECTING AN INTRAOCULAR LENS**

(71) Applicant: Lambda-X Ophthalmics, 1400 Nivelles (BE)
(72) Inventor: GATINEL, Damien, 75006 Paris (FR); STERN, Benjamin, 75006 Paris (FR); JOANNES, Luc, 1400 Nivelles (BE); VILLEMAGNE, Noé, 1400 Nivelles (BE)
(74) Representative: Calysta NV

(57) **Abstract**

System for selecting an intraocular lens (2i) to be implanted into an eye (1) comprising an eye measurer (4) and a processing means (5) configured for computing a post-surgical visual performance of the plurality of intraocular lenses (2i) in the eye (1) of the patient to output a selection advise; wherein the post-surgical visual performance computation comprises: estimating a post-surgical position of the intraocular lens (2i), generating a post-surgical optical model (OEi) of the eye (1), and simulating the post-surgical visual performance of the intraocular lens (2i); the system comprises a lens measurer (3) for measuring the plurality of intraocular lenses (2i) to obtain a plurality of lens measurements (MLi) of the intraocular lenses (2i), wherein the processing means (5) is configured for obtaining the optical lens model (OLi) of each intraocular lens (2i) measured by the lens measurement equipment (3) based on its lens measurement (MLi).

## Description

### Technical Field

The present invention relates to a method, system and computer program for selecting an intraocular lens (IOL), particularly for cataract surgery, specific to a patient's eye.

### Prior art

Cataract surgery is a common procedure aimed at replacing the natural lens affected by cataracts with a synthetic lens, also called IOL. An accurate selection of the IOL is crucial for optimizing the visual outcomes for patients, as it significantly impacts post-surgical vision quality.

Existing methodologies for IOL selection predominantly rely on pre-surgical measurements and subjective judgment. However, these approaches can fall short in addressing complex corneal irregularities, particularly in patients with an history of refractive surgeries like laser-assisted in situ keratomileusis (LASIK) or Photorefractive keratectomy (PRK). Also depend those subjective selections heavily on the experience of the surgeon.

Some patents propose systems for automatically selecting an optimal IOL utilizing an integrated approach that combines pre-surgical eye measurements with stored data for a plurality of lOLs. These systems simulate for each IOL the visual outcome based on the stored IOL parameters and the measured pre-surgical eye parameters using, for example a ray tracing simulation.

However, even if those automatic selection systems have been proposed in theory for many years, no working model has reached the market yet. This is probably because the optical lens models were oversimplified and reduced to some basic lens parameters so that the results did not correspond to the reality.

### Brief summary of the invention

It is the object of the invention to improve the theoretically proposed systems of automatic selection of intraocular lenses so that they work reliably and can actually be used in practice.

According to the invention, this object is solved by a method, a system and a computer program according to the independent claims.

According to the invention, this object is solved by a method for selecting an intraocular lens to be implanted into an eye of a patient, the method comprising the steps of: a) providing for each of a plurality of intraocular lenses an optical lens model and lens data; b) measuring with an eye measurement equipment the eye of the patient to obtain a pre-surgical eye measurement; c) generating, in a processing means, a pre-surgical optical model of the eye of the patient based on the pre-surgical eye measurement; d) computing, in the processing means, a post-surgical visual performance of a subset of the plurality of intraocular lenses in the eye of the patient; e) outputting from the processing means a selection advise for selecting the intraocular lens to be implanted in the eye of the patient based on the computed post-surgical visual performances of the plurality of intraocular lens. The step d) is realized by performing for each intraocular lens of the plurality of lenses the following steps: i) estimating a post-surgical position of the intraocular lens in the pre-surgical eye model based on the pre-surgical eye measurement and based on the lens data of the intraocular lens; ii) generating a post-surgical optical model of the eye by combining the pre-surgical optical model of the eye with the optical lens model at the estimated post-surgical position in the pre-surgical eye model; iii) simulating the post-surgical visual performance of the intraocular lens based on the post-surgical optical model of the eye. Step a) comprises the steps of: measuring the intraocular lens to obtain a lens measurement, preferably a wavefront measurement of the lens, and obtaining the optical lens model based on the lens measurement.

According to the invention, this object is solved by a system for selecting an intraocular lens to be implanted into an eye of a patient, the system comprising: an eye measurement equipment for measuring the eye of the patient to obtain a pre-surgical eye measurement and a processing means. The processing means is configured for : a) generating a pre-surgical optical model of the eye of the patient based on the pre-surgical eye measurement; b) for computing a post-surgical visual performance of the plurality of intraocular lenses in the eye of the patient, and c) for outputting a selection advise for selecting the intraocular lens to be implanted in the eye of the patient based on the computed post-surgical visual performances of the plurality of intraocular lenses. The post-surgical visual performance of the plurality of intraocular lenses in the eye of the patient are computed by performing for each intraocular lens of the plurality of intraocular lenses the following steps: i) estimating a post-surgical position of the intraocular lens in the pre-surgical eye model based on the pre-surgical eye measurement and based on lens data of the intraocular lens; ii) generating a post-surgical optical model of the eye by combining the pre-surgical optical model of the eye with an optical lens model at the estimated post-surgical position in the pre-surgical eye model; and iii) simulating the post-surgical visual performance of the intraocular lens based on the post-surgical optical model of the eye. The system comprises further a lens measurement equipment for measuring the plurality of intraocular lenses to obtain a plurality of lens measurements of the intraocular lenses. The processing means is configured for obtaining the optical lens model of each intraocular lens measured by the lens measurement equipment based on its lens measurement.

According to the invention, this object is solved by a (transitory or non-transitory) computer program for selecting an intraocular lens to be implanted into an eye of a patient. The computer program comprising a set of instruction which, when executed on a processing means, perform the following steps: a) storing for each of a plurality of intraocular lenses an optical lens model and lens data; b) receiving a pre-surgical eye measurement of the eye of the patient; c) generating a pre-surgical optical model of the eye of the patient based on the pre-surgical eye measurement; d) computing the post-surgical visual performance of the plurality of intraocular lenses in the eye of the patient; and e) outputting a selection advise for selecting the intraocular lens to be implanted in the eye of the patient based on the computed post-surgical visual performances of the plurality of intraocular lenses. The step d) is realized by performing for each intraocular lens of the plurality of intraocular lenses the following steps: i) estimating a post-surgical position of the intraocular lens in the pre-surgical eye model based on the pre-surgical eye measurement and based on the lens data of the intraocular lens; ii) generating a post-surgical optical model of the eye by combining the pre-surgical optical model of the eye with the optical lens model at the estimated post-surgical position in the pre-surgical eye model; and iii) simulating the post-surgical visual performance of the intraocular lens based on the post-surgical optical model of the eye. The stored optical lens models of the intraocular lenses are obtaining based on lens measurements of the respective intraocular lenses.

By determining the optical lens models of the intraocular lenses based on measurements of the intraocular lenses, the optical lens models can be determined with a high precision and independently from any information of the lens manufacturers. The lens measurement allows to generate optical lens models even for complex lenses and represent their optical behaviour with a low error. Existing methods are based normally on the parameters available from the manufacturers which oversimplify the optical model. Detailed lens models are only available to the lens manufacturers themselves and would limit the lens selection to a single lens manufacturer. Thus, the invention allows to increase the quality of the optical lens models for intraocular lenses independently from the lens manufacturer allowing thus to offer a selection based on a highly reliable visual performance simulation for many different manufacturers. In addition, the resulting visual performance of different intraocular lenses becomes comparable as they can be obtained by the same type of lens measurement, while before they were based on information from the lens manufacturer which provided sometimes very precise models (or many parameters) and sometimes very poor models. When combining the lens measurement with measurements of the eye of the patient representing also complex corneas topographies, the generated post-surgical eye model can predict very well the post-surgical eye of the patient. This allows surgeons to obtain an unbiased and reliable selection advise for the optimal intraocular lens for a specific patient, even for patients with very complex corneas.

The dependent claims refer to further advantageous embodiments.

In one embodiment, the optical lens model is obtained by ray tracing simulation. The use of ray tracing simulations allows to obtain high quality optical lens models from lens measurements. This can be obtained either by an inverse ray tracing simulation to obtain the optical model (fully) from the lens measurement, preferably a wavefront measurement of the lens, or by verifying the correctness of an optical lens model received with a ray tracing simulation to verify, if the ray tracing simulation through the optical model received corresponds (within a certain error margin) to the lens measurement.

In one embodiment, the post-surgical position of the intraocular lens is estimated with a formula based on a thick lens model or based on a thick lens pseudophakic eye model. Not only the correct model, but also the correct position of the intraocular lens in the post-surgical eye is an important parameter to obtain the correct prediction for the visual performance of an intraocular lens. By using a thick lens model for obtaining the position of the intraocular lens, the prediction precision has been significantly increased which proved in combination with the lens measurements to improve significantly the resulting visual performance of the intraocular lenses. Also, the thick lens model which distinguishes between the anatomic and optical position of the intraocular lens allows to estimate first the anatomical position of the intraocular lens and then use the detailed lens measurement for obtaining the precise optical position (position of one of the principal planes of the thick lens) of the intraocular lens.

In one embodiment, the power of the intraocular lens to be implanted is determined based on the estimated post-surgical position of the intraocular lens and based on a desired power of the eye, wherein the post-surgical optical model of the eye is generated by inserting in the pre-surgical optical model of the eye at the estimated post-surgical position the optical lens model of the intraocular lens for the determined power of the intraocular lens. While most power formulas determine directly the power of the intraocular lens, it is most advantageous to determine (independently from the power) first the position of the intraocular lens, because the position can be used first for determining the power and second for positioning the optical lens model correctly in the post-surgical eye model.

In one embodiment, the post-surgical position of the intraocular lens is computed based on the pre-surgical eye measurement and based on the historical data of the intraocular lens in previous patients and based on the lens data.

In one embodiment, the post-surgical position of the intraocular lens is computed based on an artificial intelligence (Al) engine which receives the pre-surgical eye measurement and information about the intraocular lens and outputs the post-surgical position, wherein the Al engine is trained by the historical data which comprise for each previous patient eye a pre-surgical eye measurement, an intraocular lens implanted and an annotated post-surgical position of the respective intraocular lens implanted in the previous patient eye.

In one embodiment, the annotated post-surgical position is calculated using a thick lens pseudophakic eye model from the post-surgical measurement of the effective power/refraction of the eye. Thus, the thick lens model allows to generate sufficient training data for intraocular lenses for which the anatomic position of the lens was not measured post-surgically. Due to the use of the thick lens which distinguishes between the anatomic and optical position of the intraocular lens, the training data can be computed with sufficient quality from the measurement of the power of the post-surgical eye.

In one embodiment, the estimated post-surgical position of the intraocular lens is the anatomic position or the optical position of the intraocular lens along the optical axis of the eye in a thick lens pseudophakic eye model in which the optical position is different from the anatomical position of the intraocular lens.

In one embodiment, the pre-surgical eye measurement comprises biometric data of the eye and the topographic data of the cornea. The topography of the cornea allows to generate a detailed optical eye model of the cornea which will also play an important role in the post-surgical eye model. The topography of the eye will allow to represent also complex cornea shapes like after LASIK, PRK, and other interventions on the eye. Preferably, the topography data contain the topography of the anterior cornea surface and the topography of the posterior cornea surface and the cornea in the pre-surgical eye model and/or in the post-surgical eye model represents the topography of the anterior and posterior cornea surface measured. This improves the quality of the visual performance of the eye with the implanted intraocular lens, especially when considering a thick lens model for the cornea.

In one embodiment, the topographic data of the cornea are measured with a topographer and/or with an optical coherence tomographer.

In one embodiment, the post-surgical visual performance of the intraocular lens is simulated based on a ray tracing simulation of the post-surgical optical model of the eye. Once the post-surgical optical eye model has been generated, the ray tracing simulation allows to simulate the visual performance for optical eye models of any complexity.

In one embodiment, the post-surgical visual performance of the intraocular lens is measured from a simulated wavefront measurement based on the post-surgical optical model of the eye, preferably based on a ray tracing simulation of the post-surgical optical model of the eye.

In one embodiment, the optical lens models of the plurality of intraocular lenses are stored in a database.

Other embodiments according to the present invention are mentioned in the appended claims and the subsequent description of an embodiment of the invention.

### Brief description of the Drawings

Fig. 1 shows a schematic illustration of the pre-surgical anatomy of an eye of a patient with eye measurement parameters.
Fig. 2 shows the schematic illustration of the pre-surgical anatomy of the eye of the patient with eye measurement parameters.
Fig. 3 shows a flow diagram of the method for selecting an IOL according to the invention.
Fig. 4 shows an embodiment of providing the lens model of the IOL.
Fig. 5 shows an embodiment for iteratively computing a post-surgical visual performance.
Fig. 6 shows an embodiment of the system for selecting an IOL according to the invention.
Fig. 7 shows an example of a thick lens pseudophakic eye model of the post-surgical eye.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

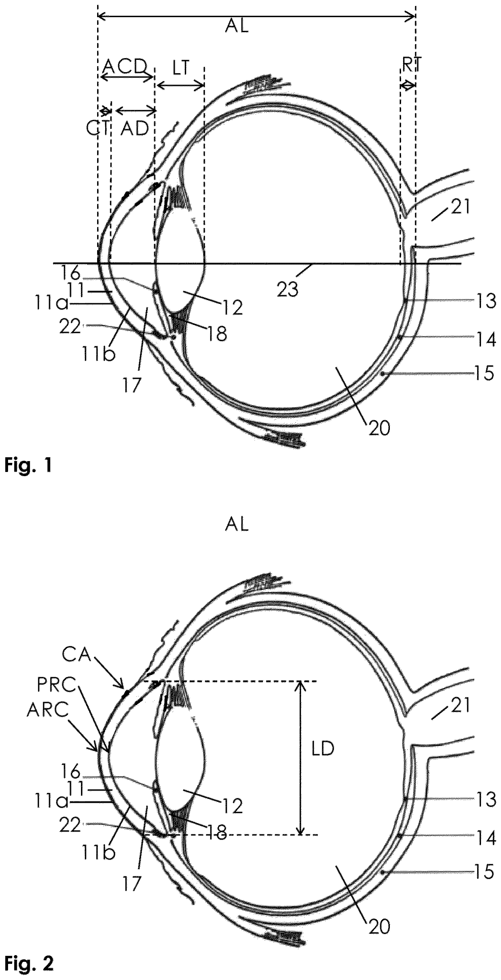

Fig. 1 and 2 show the anatomy of the eye or eyeball 1 before surgery (pre-surgical). The parts of the eyeball 1 will shortly be described. The cornea 11 is the clear outermost layer of the eye 1 that covers the iris 16, pupil, and anterior chamber 17. It plays a vital role in focusing light onto the lens 12. The iris 16 is the colored part of the eye that surrounds the pupil and controls the amount of light entering the eye 1 by adjusting the size of the pupil. The pupil is the black circular opening at the center of the iris 16, which regulates the amount of light entering the eye 1. The lens 12 is a clear, flexible structure located behind the iris 16 and pupil, which helps to focus light onto the retina 13. The retina 13 is the layer of light-sensitive cells at the back of the eye 1 that converts incoming light into electrical signals that are sent to the brain via the optic nerve 21. It contains two types of photoreceptor cells called rods and cones, which respond to different levels of light and color. The optic nerve 21 is a bundle of nerve fibers that carries the electrical signals from the retina 13 to the brain, where they are interpreted and processed into visual images. The sclera 15 is the white outer layer of the eye 1 that protects the eyeball and maintains its shape. The choroid 14 is the layer of blood vessels between the sclera 15 and the retina 13 that provides oxygen and nutrients to the eye 1. The ciliary body 22 is a ringshaped muscle that controls the shape of the lens and helps to adjust focus. The aqueous humor is a clear fluid that fills the front part of the eye, especially the anterior chamber 17 and helps to maintain its shape and pressure. The vitreous humor or vitreous body 20 is a clear gel-like substance that fills the back part of the eye and helps to maintain its shape and support the retina 13. The posterior chamber 18 is arranged between the iris 16 and the ciliary body 22. The cornea 11 and lens 12 work together to bend and focus incoming light onto the retina 13 at the back of the eye 1. The ciliary body 18 and lens 12 work together to adjust the focus of the eye 1, allowing us to see objects at different distances. The optical axis 23 indicates the optical axis of the eye 1. The adjective "anterior" describes the direction towards the cornea 11 along the optical axis 23, while the adjective "posterior" describes the direction towards the retina 13 along the optical axis 23. For example, the anterior cornea surface 11a describes the outer surface of the cornea 11, while the posterior cornea surface 11b describes the inner surface of the cornea 11.

Fig. 1 shows the common biometric data of the eye 1: axial length AL of the eyeball 1, corneal thickness CT, anterior chamber depth ACD, lens thickness LT. Fig. 2 shows further biometric data like anterior corneal curvature radius ARC (apical or average), posterior corneal curvature radius PRC (apical or average), corneal diameter LD (also called limbus diameter or white to white diameter).

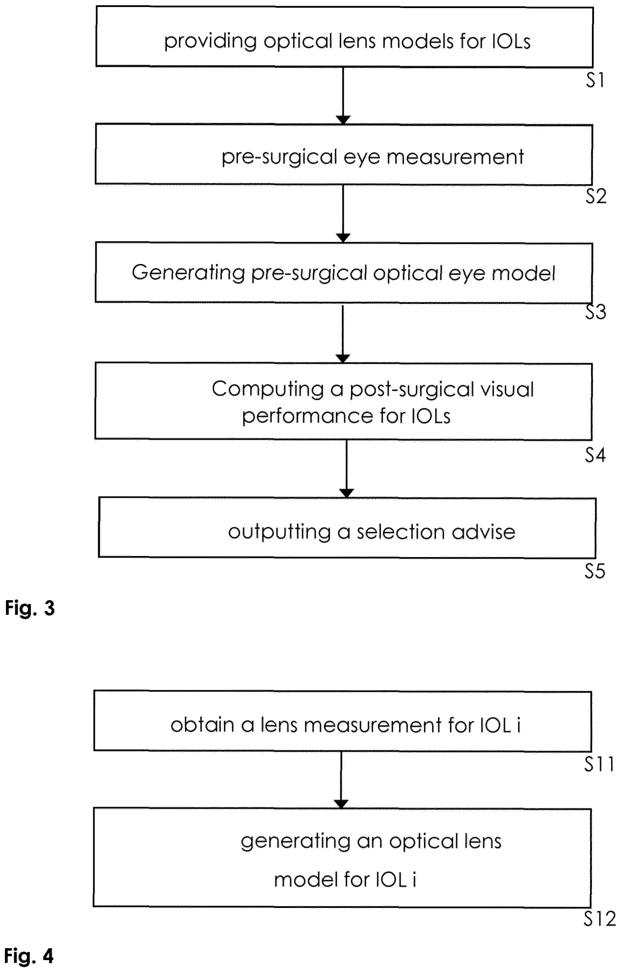

Fig. 3 shows an embodiment for a method according to the invention for selecting an IOL 2 among a plurality of IOLs 2i (with i=1, ..., N) for the eye 1 of a patient.

In step S1, an optical lens model OLi for each of a plurality of lOLs 2i is provided. Thus, the method provides for each of the plurality of lOLs 2i an optical model OLi with i=1, ..., N. The optical lens model OLi shall refer to the optical lens model of the i-th IOL 2i. Thus, for N IOLs, the method provides N (different) OLi with i=1, ...., N. The IOLs 2i comprise preferably lOLs of different manufacturers. Preferably, each IOL 2i refers to a certain IOL type which can be ordered with (Ni) different power values Pi,j with j=1, ..., Ni. Preferably, then the plurality of optical lens models contain one optical lens model OLij for each type i of IOL with i=1,..., N and for each power value of the respective type i with j=1, ..., Ni. For some types 2i of IOLs, only one power is available, i.e. Ni would then be 1. Preferably, the optical lens models OLi or OLij are stored in a storage means 6a so that the optical lens model OLi can be accessed quickly on the storage means 6a.

Fig. 4 shows one preferred embodiment for providing the optical lens model OLi. The following process is preferably applied for each or at least for most of the plurality of optical lens models OLi.

In step S11, a lens measurement MLi of the IOL 2i is performed. The lens measurement MLi is configured to generate an optical lens model OLi for the IOL 2i based on the lens measurement MLi. The lens measurement MLi characterizes the IOLi. The lens measurement MLi is preferably a wavefront measurement. The lens measurement MLi is preferably obtained by a wavefront measuring device or instrument 3. The measuring device 3 is preferably an interferometer, preferably uses a Mach-Zehnder interferometric setup. The interferometer, especially the specific interferometer technology works particularly well for getting a precise wavefront measurement of the IOL 2i. However, also other measurement technologies can be used. Preferably, the different lens measurements MLi are performed preferably with the same measurement technology, preferably with the same measurement instrument 3. The same measurement instrument 3 shall mean here the same type of instrument (same manufacturer, same measurement hardware, e.g. same model), but shall allow to use different examples of the same type of measurement hardware for measuring the different IOLs. If an IOL of type i exist with different power values j, the lens measurement MLi,j is performed for each power value Pj with j=1,..., Ni.

In step S12, the optical lens model OLi for the IOL 2i is generated based on the lens measurement MLi of the IOL 2i. The optical lens model OLi is preferably generated in a processing means 5. Preferably, the optical lens model OLi is determined based on ray tracing simulation/modelling. Preferably, the optical lens model OLi is obtained by inverse ray tracing simulation from the lens measurement MLi, preferably from the wavefront measurement MLi. In an inverse ray tracing simulation, the optical lens model OLi is iteratively changed until the ray tracing of the optical lens model OLi results in the lens measurement MLi (within a threshold or tolerated error range). However, it is also possible that the OLi is received (e.g. from the manufacturer) and is verified with a ray tracing simulation of the received optical lens model OLi to result in the measured lens measurement MLi (within a threshold or tolerated error range). The verification includes preferably a computation of the error between the simulated lens measurement or wavefront MLi (received error) based on the received OLi and the lens measurement MLi actually measured. If this error is below a certain threshold, it can be accepted. The error of the received optical lens model can also be compared with an (optimized) error of a second optical model (the optimized optical lens model) obtained by inverse ray tracing optimization based on the measured lens measurement MLi. The optimized error is thus obtained by the difference of the simulated lens measurement based on the second optical model and of the lens measurement MLi actually measured. If the received error of the received optical lens model is lower, the received optical lens model is used, if the optimized error of the optimized optical lens model is lower, the optimized optical lens model is used. If an IOL of type i exist with different power values j, an optical lens model OLi,j is determined for each power value Pj based on the respective lens measurement MLi,j with j=1,..., Ni.

Obtaining the optical lens model OLi based on the lens measurement MLi has a number of strong advantages over the prior art. The optical lens models OLi are not only based on some few (normally linear) parameters available from the manufacturer and the optical lens models OLi can be determined for any manufacturer without depending on the information from the manufacturer. Especially, the optical models OLi of different lOLs become better comparable as they have been all determined by a lens measurement ML, especially when they were measured with the same lens measurement instrument 3.

Going back to the method of Fig. 3, step S1 provides for each of the plurality of lOLs i or i,j an optical lens model OLi or OLij.

In step S2, a pre-surgical eye measurement ME of the eye 1 of the patient is performed. In other words, the eye 1 of the patient is measured before surgery for implanting an IOL in the eye 1 of the patient resulting in the eye measurement ME. The eye measurement ME includes preferably biometric data of the eye 1 and topography data of the cornea 11 of the eye 1.

The topography data contain preferably the topography of the anterior cornea surface 11a and the topography of the posterior cornea surface 11b. The topography data are preferably representing a complex topography, i.e. a non-parametrized topography. The topography data are represented preferably in the form of a point cloud. The point cloud is measured/stored in one dimension (e.g. the topography curve in the radial direction in case of radial symmetry showing the topographical height of the cornea surface for each point along a radius vector extending from the optical axis 23 radially outwards), but preferably in two dimensions (two dimensional topography showing a topography map) in order to represent also radial asymmetries. The topography data can be for example measured with corneal topographer, optical coherence tomography (OCT) instruments or combined biometers.

The biometric data comprise preferably one or more or all of the axial length AL of the eyeball 1, the corneal thickness CT, the anterior chamber depth ACD, the lens thickness LT, the anterior corneal curvature radius ARC (apical or average), the posterior corneal curvature radius PRC (apical or average), the corneal diameter LD. Further potential biometric data could be pupil position, variations in pupil diameter (pupil dynamics), and/or the kappa angle (which reflects the relative decentration of the optical surfaces). These data are obviously not exclusive and further data can be used. Some of those parameters could be replaced by other parameters. The biometric data are normally measured with a biometer (instrument).

The eye measurement ME is preferably measured with an eye measurement equipment/instrument 4. The eye measurement equipment 4 can be a single instrument which measures all eye measurement data ME (like a combined biometer) or could comprise two or more eye measurement instruments like a biometer and a topographer to measure them.

The eye measurement ME comprises preferably a set of eye model parameters and a set of IOL parameters. The set of eye model parameters are used to compute a presurgical eye model. The set of eye model parameters comprise the topographical data of the cornea 11. The set of eye model parameters comprise further biometric data necessary to establish the presurgical model of the eye 1 like the axial length AL and/or the corneal thickness CT. The presurgical model OE of the eye 1 could leave out the lens 12 as the lens 12 is anyway replaced by the IOL 2 in one of the next steps. The set of IOL parameters of the eye measurement parameters ME are parameters used to estimate the post-surgical position of the IOL 2 and/or the power of the IOL 2 as will be explained below. Some parameters of the eye measurement ME could be in the set of eye model parameters and in the set of IOL parameters, i.e. could be used for modelling the presurgical eye 1 and for computing the post-surgical position and/or power of the IOL 2.

In step S3, a pre-surgical optical eye model OE is generated (in the processing means 5) based on the eye measurement ME. The pre-surgical optical eye model OE is generated based on the set of eye model parameters. The form of the cornea 11, preferably of the anterior cornea surface 11a and of the posterior cornea surface 11b is generated based on the topographic data. The form of the cornea 11 could use additionally the biometric data of the cornea thickness CT. The position of the retina 13 relative to the cornea 11 could be modelled based on the axial length AL. Further parameters could be used to further improve the pre-surgical eye model OE.

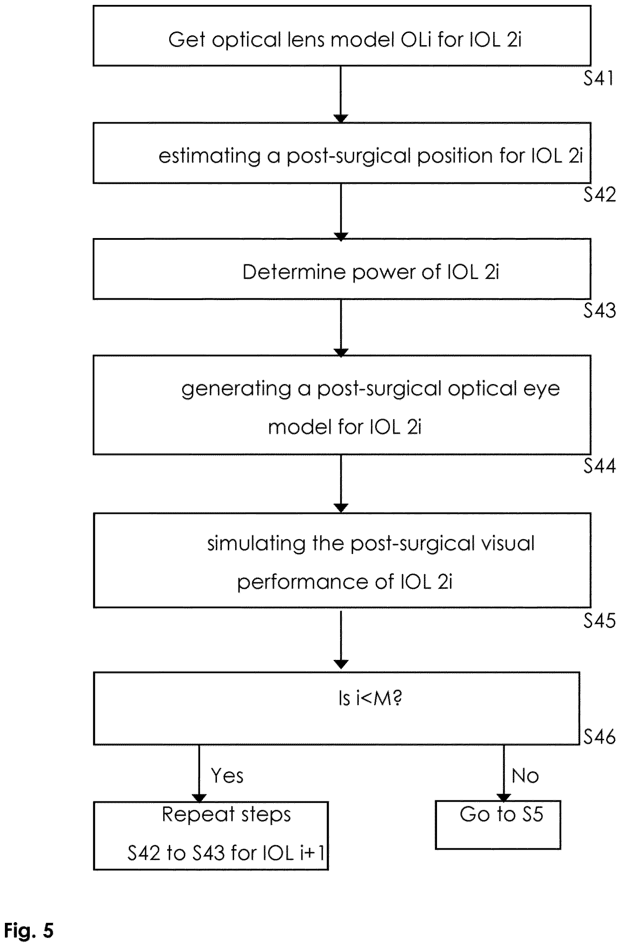

In step S4, a post-surgical visual performance of a subset of the plurality of intraocular lenses IOLs 2 in the eye of the patient is computed. The computation is performed based on the eye measurement ME and based on the optical lens models OLi of the plurality of intraocular lenses 2i. This will be described in more detail with the help of Fig. 5.

The steps S41 to S44 are repeated for each IOL 2i of the plurality of IOLs 2. The M IOLs 2 for which the step S4 is performed can be the same as the number N of IOLs 2 stored in the system (i.e. the performance value is calculated for each IOL 2). In another embodiment, M is smaller than N, i.e. the performance value is calculated only for a subset of M IOLs 2 of the N available IOLs 2. The subset of M IOLs 2 can be chosen based on different criteria like availability (IOLs 2 currently not available for surgery can be excluded from the simulation as they can anyway not be used) or suitability for the patient. The suitability can refer to the pathology of the patient like a short-sighted patient, it is not necessary to simulate the performance of long-sight IOLs 2. For simplicity, the plurality of IOLs 2 as used in the claims refers to the lOLs 2 used to compute a performance value of these IOLs 2. In the following, the computation is explained exemplary for one IOL 2i of the M IOLs. This computation is iteratively repeated for each IOL 2i with the iteration i being 1, 2, ..., or M. The iterations can happen at any order and can happen also at the same time as each iteration i is independent from the other iterations.

In step S41, the optical lens model OLi and the lens data of the IOL 2i of this iteration i is retrieved, preferably from a storage 6a. The lens data of the IOL 2i can be stored separately or could also be retrieved from the optical lens model OLi.

In step S42, the post-surgical position of the IOL 2i of this iteration i (subsequently abbreviated for the description of S42 with "the position") is determined based on the eye measurement ME, based on the lens data of the IOL 2i of this iteration i. The position refers at least to the position along the optical axis 23 of the eye 1 between the cornea 11 and the retina 13 (basically a position in one dimension).

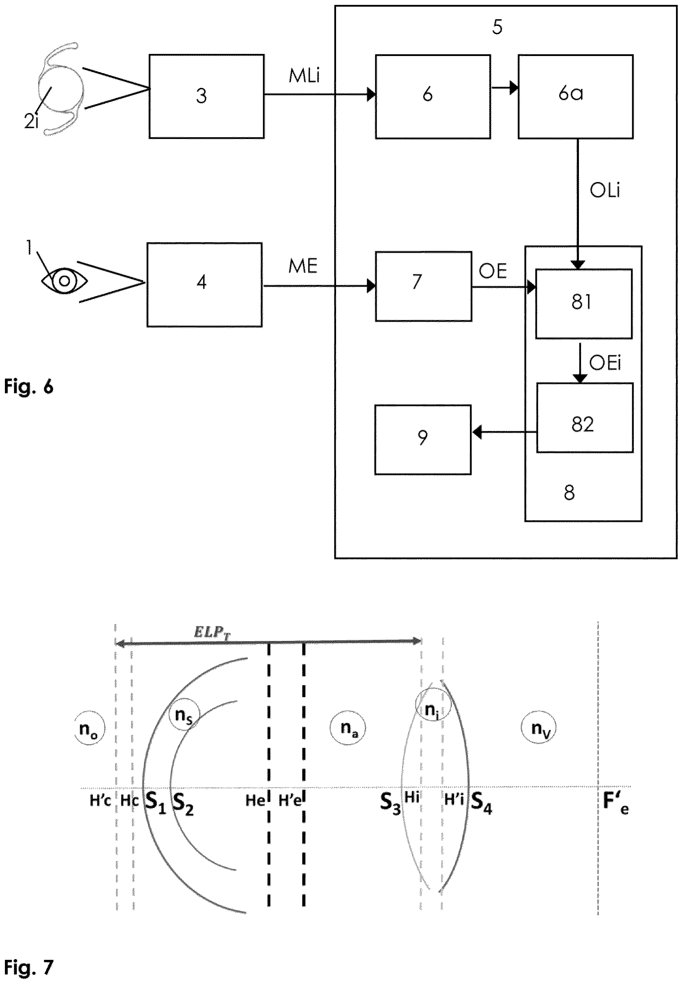

The position is estimated preferably using a thick lens model of the lenses of the eye, in particular of the IOL 2 and/or of the cornea 11. Thin lens models and thick lens models are well known terms in optics. Thin lens models use an approximation which holds for thin lenses. Thick lens models are more complex and must be used, when the lenses become thicker. For example, a thick lens model comprises for the thick lens two principal planes, while a thin lens model has only one principal plane (normally the center plane of the lens). Especially in thick lens models, the anatomical position of the thick lens is well distinguished from the optical or (one of the) principal plane(s) of the lens, while in a thin lens model this is the same. The term thick lens model is thus not relative, but binary as it distinguishes only two types of optical lens models. The position is estimated based on a thick lens pseudophakic eye model. The thick lens pseudophakic eye model models the post-surgical eye as a thick IOL 2 and a thick cornea 11. Fig. 7 shows such a model. Fig. 7 shows the anterior principal plane H'c and the posterior principal plane He of the cornea 11, the anterior corneal position S1 (on the optical axis 23) of the anterior corneal surface 11a, the posterior corneal position S2 (on the optical axis 23) of the posterior corneal surface 11b, the anterior principal plane Hi and the posterior principal plane H'i of the IOL 2, the anterior IOL position S3 (on the optical axis 23) of the anterior IOL surface, the posterior IOL position S4 (on the optical axis 23) of the posterior IOL surface, the refractive index n0 of air, the refractive index ns of the cornea 11, the refractive index na of the anterior chamber 17, the refractive index ni of the IOL, the refractive index nv of the vitreous body 20. It can be clearly seen that the anatomical position S3 of the IOL is different from the optical position Hi. The effective principal planes He and H'e and the effective focal point F'e defines an effective thick lens representing the optical system of the combination of the corneal thick lens 11 and the thick lens IOL 2.

An IOL is injected in the eye and finds itself its position. The anatomical position is therefore preferably predicted by a statistical approach. Preferably, the anatomical position of the IOL 2i of this iteration i is determined based on historical data of this IOL 2i, preferably based on an artificial intelligence (Al) engine. The Al engine gets the set of IOL parameters of the eye measurement ME, i.e. the parameters of the eye measurement ME used for determining the IOL position, and the IOL 2i of this iteration i as input and outputs the estimated position of the IOL 2i of this iteration i. The set of IOL parameters are for example axial length AL of the eyeball 1, corneal thickness CT, anterior chamber depth ACD, lens thickness LT, corneal diameter LD. The position output is preferably the anatomical position of the IOL 2i.

The Al engine is trained with historical data of eyes in which the IOL 2i of this iteration i were implanted. The training data contains for each of those eyes, the same pre-surgical input features/parameters (set of IOL parameters + IOL 2i), i.e. the eye measurement ME done before the surgery. In addition, the training data contains for each of those eyes with the IOL 2i implanted the achieved position (annotated position) of the IOL 2i implanted. The annotated position can be input with an error parameter like a standard deviation or variance. The annotated position can be measured post-surgical. However, since this is rarely done, an alternative to obtain the annotated position could be to compute from the achieved optical effective focus/refraction of the eye which is measured post-surgically the (preferably anatomic) position of the IOL 2 using the thick lens pseudophakic eye model described above. Thus, if the

The Al model can be a simple regression model. The Al model can be a machine learning model or even more complex models like neuronal network.

If the output of the Al engine yields the anatomical position of the IOL 2i of this iteration I, the thick lens pseudophakic eye model described above can be used to calculate the optical position Hi of the IOL 2i. In an alternative Al engine, the optical position could be output directly from the Al engine.

In step S43, the power of the IOL 2i is determined based on the (preferably optical) position of the IOL 2i determined in step S42 and based on the desired power/refraction of the eye 1 after the surgery. Once the position of the IOL 2i is known, the power of the IOL 2i can be calculated based on the thick lens pseudophakic eye model. The IOL2ij exists with Ni different power values Pij with j=1, ..., Ni and the power Pij is selected which comes closest to the desired power/refraction of the eye 1, i.e. the closest to effective focal point Fe. Alternatively, it would also be possible to determine the power of the IOL 2i with a formula not using the position of the IOL 2i. In this case, the step S43 could be performed also before the step S42.

In step S44, a post-surgical optical eye model OEi with the IOL 2i of this iteration is generated by combining the pre-surgical model of the eye OE with the optical lens model OLi for the IOL 2i (with the power determined in step S43). The optical lens model OLi for the IOL 2i (with the power Pj determined in step S43) is inserted in the pre-surgical eye model OE at the position determined in step S42. The steps S3 of generating a pre-surgical eye model and S44 of combining the pre-surgical eye model with the optical lens model OLi of the IOL 2i of this iteration i shall include also a combined step which generates the post-surgical optical eye model OEi with the IOL 2i of this iteration i directly based on the eye measurement ME and the optical lens model OLi. In this case, the generation of the parts of the post-surgical optical eye model OEi corresponding to the pre-surgical eye model, e.g. the cornea 11 and/or the retina 13 correspond to step S3.

In step S45, the post-surgical visual performance of the IOL 2i of the iteration i is simulated based on the post-surgical eye model OEi. The visual performance value, also called often optical quality metric, can be for example one or more of the Strehl Ratio and the through-focus Modulation Transfer Function (MTF). Obviously, other quality metrics are possible. Preferably, the simulation is done by a ray tracing simulation. Since the topography of the cornea 11 of the post-surgical eye model OEi is based on detailed eye measurements ME and the IOL 2i of the post-surgical eye model OEi is based on a detailed lens measurement MLi, the post-surgical visual performance of the IOL 2i implanted in the eye can be estimated with a high precision. Due to the lens measurements ML of the IOLs, the post-surgical visual performances of different IOLs 2 become comparable as the optical lens models OL were obtained based on the same type of lens measurement (with the same degree of precision). Especially, combined with the use of the thick lens model for estimating the position of the IOL 2i (and its power) with a high precision further significantly improves post-surgical visual performance, as errors in the position of the IOL can be reduced and thus the error in the visual performance reduced.

The steps S41 to S45 are repeated for each IOL 2i (indicated in step S46) until the visual performance for each IOL 2i of the N lOLs of the plurality of IOLs 2 are computed.

Thus, step S4 results in one or more visual performance value(s) for each IOL 2i of the plurality of IOLs 2.

In step S5, a selection advise for selecting the intraocular lens to be implanted in the eye 1 of the patient is determined/output based on the computed post-surgical visual performances of the plurality of intraocular lens. The selection advise could be the output of the IOL 2 or a number of best IOLs 2 based on the performance values. The selection advise could also be a list of the plurality of IOLs 2 with their calculated performance value. This list could be sorted by the performance value so that the lOLs 2 with the best performance values are clearly visible. The steps S4 and S5 could also be performed together, e.g. by memorizing only the IOL or IOLs with the best performance value(s) from one iteration to the next.

The method of Fig. 3 is preferably repeated for each eye of the patient (or in case of only one pathologic eye only for the pathologic eye). For each eye, there might be another IOL 2i recommended in step S5.

Fig. 6 shows an exemplary embodiment of a system according to the invention. The system comprises a lens measurement equipment 3, an eye measurement equipment 4 and a processing means 5.

The lens measurement equipment 3 is configured to perform the lens measurement MLi of the IOLs 2i. The lens measurement equipment 3 is configured to perform the step S11. The lens measurement MLi is preferably a wavefront measurement. The lens measurement MLi is preferably obtained by a wavefront measurement device or instrument 3. The lens measurement device 3 is preferably an interferometer, preferably uses a Mach-Zehnder interferometric setup. The interferometer, especially the specific interferometer technology works particularly well for getting a precise wavefront measurement of the IOL 2i. However, also other measurement technologies can be used. Preferably, the different lens measurements MLi for different IOLs 2i are performed preferably with the same measurement technology, preferably with the same measurement instrument 3. The same measurement instrument 3 shall mean here the same type of instrument (same manufacturer, same measurement hardware, e.g. same model), but shall allow to use different examples of the same type of measurement hardware for measuring the different IOLs. If an IOL of type 2i exist with different power values j, the lens measurement MLi,j is performed for each power value Pj with j=1,..., Ni.

The eye measurement equipment 4 is configured to perform a pre-surgical eye measurement ME of the eye 1 of the patient. The eye measurement equipment 4 is configured to perform step S2. The eye measurement ME includes preferably biometric data of the eye 1 and topography data of the cornea 11 of the eye 1. The eye measurement equipment 4 can be a single instrument which measures all eye measurement data ME (like a combined biometer) or could comprise two or more eye measurement instruments like a biometer and a topographer to measure them with two instruments.

The processing means 5 is configured to perform the steps S3, S4, S5, S12, S41, S42, S43, S44, S45 and/or S46. For the sake of brevity, the description of these steps is not repeated here. The processing means 5 can be realized in one computing device or can be distributed over different computing devices. The processing means 5 can be included in the eye measurement device 4 or in distinct computing device. The distinct computing device can be arranged remote from the eye measurement equipment 4 and/or remote from the lens measurement equipment 3. The (distinct) computing device can be a server. The distinct computing device, the server or the processing means 5 receives from the lens measurement equipment 3 the lens measurement MLi for the IOLs 2i and receives from the eye measurement equipment 4 the eye measurement 4. The processing means 5 can also be distributed over two or three of the eye measurement equipment 4, the lens measurement equipment 3 and the computing device/server. For example, the step S12 for computing the lens model OLi based on the lens measurement MLi can be realized in the lens measurement equipment 3 so that the lens measurement equipment 3 sends the optical lens model OLi to the computing device. For example, the step S3 for generating the pre-surgical eye model OE based on the eye measurement ME can be realized in the eye measurement equipment 4 so that the eye measurement equipment 4 sends the optical eye model OE to the computing device. The processing means 5 comprises preferably a server. Preferably, all the above steps are performed on the server. However, it is for example possible that some of the steps mentioned above, or parts of those steps are performed on a client device arranged preferably in the vicinity of the eye measurement device (or directly in the eye measurement device 4) and/or that some of the steps mentioned above, or parts of those steps are performed on a client device arranged preferably in the vicinity of the lens measurement device (or directly in the lens measurement device 3). The server has preferably a user management wherein each user can access the server based on user credentials. The user can preferably upload one (or two) eye measurements ME and obtains from the server back the IOL selection advice of step S5.

The computer program of the invention corresponds basically to the steps performed by the processing means 5.

It should be understood that the present invention is not limited to the described embodiments and that variations can be applied without going outside of the scope of the claims.

## Claims

1. Method for selecting an intraocular lens (2i) to be implanted into an eye (1) of a patient, the method comprising the steps of:
providing for each of a plurality of intraocular lenses (2i) an optical lens model (OLi) and lens data;
measuring with an eye measurement equipment (4) the eye (1) of the patient to obtain a pre-surgical eye measurement (ME);
generating, in a processing means (5), a pre-surgical optical model of the eye (1) of the patient based on the pre-surgical eye measurement (ME);
computing, in the processing means (5), a post-surgical visual performance of a subset of the plurality of intraocular lenses (2i) in the eye (1) of the patient by performing for each intraocular lens (2i) of the plurality of lenses the following steps:
estimating a post-surgical position of the intraocular lens (2i) in the pre-surgical eye model based on the pre-surgical eye measurement (ME) and based on the lens data of the intraocular lens;
generating a post-surgical optical model (OEi) of the eye (1) by combining the pre-surgical optical model of the eye (1) with the optical lens model (OLi) at the estimated post-surgical position in the pre-surgical eye model;
simulating the post-surgical visual performance of the intraocular lens (2i) based on the post-surgical optical model (OEi) of the eye (1);
outputting from the processing means (5) a selection advise for selecting the intraocular lens (2i) to be implanted in the eye (1) of the patient based on the computed post-surgical visual performances of the plurality of intraocular lenses (2i);
**characterized in that** providing the optical lens model (OLi) comprises the following steps:
measuring the intraocular lens (2i) to obtain a lens measurement (MLi); and
obtaining the optical lens model (OLi) based on the lens measurement (MLi).

2. Method according to claim 1, wherein the optical lens model (OLi) is obtained by ray tracing simulation.

3. Method according to claim 2, wherein the optical lens model (OLi) is obtained by inverse ray tracing simulation.

4. Method according to claim 2, wherein the optical lens model (OLi) is obtained by the following steps:
receiving an optical lens model (OLi) for the intraocular lens; and
verifying the optical lens model (OLi) by ray tracing simulation based on the lens measurement (MLi).

5. Method according to any of claims 1 to 4, wherein the post-surgical position of the intraocular lens (2i) is estimated with a formula based on a thick lens model.

6. Method according to any of claims 1 to 5, wherein the power of the intraocular lens (2i) is determined based on the estimated post-surgical position of the intraocular lens (2i) and based on a desired power of the eye (1), wherein the post-surgical optical model (OEi) of the eye (1) is generated by inserting in the pre-surgical optical model of the eye (1) at the estimated post-surgical position the optical lens model (OLi) of the intraocular lens (2i) for the determined power of the intraocular lens.

7. Method according to claim 5 or 6, wherein the post-surgical position of the intraocular lens (2i) is computed based on the pre-surgical eye measurement (ME) and based on the historical data of the intraocular lens (2i) in previous patients and based on the lens data.

8. Method according to claim 7, wherein the post-surgical position of the intraocular lens (2i) is computed based on an Al engine which receives the pre-surgical eye measurement (ME) and information about the intraocular lens (2i) and outputs the post-surgical position, wherein the Al engine is trained by the historical data which comprise for each previous patient eye an pre-surgical eye measurement (ME), an intraocular lens (2i) implanted and an annotated post-surgical position of the respective intraocular lens (2i) implanted in the previous patient eye.

9. Method according to any of claims 1 to 8, wherein the pre-surgical eye measurement (ME) comprises biometric data of the eye (1) and the topographic data of the cornea.

10. Method according to claim 9, wherein the topographic data of the cornea are measured with a topographer and/or with an optical coherence tomographer.

11. Method according to one of claims 1 to 10, wherein the post-surgical visual performance of the intraocular lens (2i) is simulated based on a ray tracing simulation of the post-surgical optical model (OEi) of the eye (1).

12. Method according to one of claims 1 to 11, wherein the post-surgical visual performance of the intraocular lens (2i) is measured from a simulated wavefront measurement based on the post-surgical optical model (OEi) of the eye (1), preferably based on a ray tracing simulation of the post-surgical optical model (OEi) of the eye (1).

13. Method according to one of claims 1 to 12, wherein the optical lens models (OLi) of the plurality of intraocular lenses (2i) are stored in a database.

14. System for selecting an intraocular lens (2i) to be implanted into an eye (1) of a patient, the system comprising:
an eye measurement equipment (4) for measuring the eye (1) of the patient to obtain a pre-surgical eye measurement (ME);
a processing means (5)
for generating a pre-surgical optical model of the eye (1) of the patient based on the pre-surgical eye measurement (ME),
for computing a post-surgical visual performance of the plurality of intraocular lenses (2i) in the eye (1) of the patient, and
for outputting from the processing means (5) a selection advise for selecting the intraocular lens (2i) to be implanted in the eye (1) of the patient based on the computed post-surgical visual performances of the plurality of intraocular lenses (2i);
wherein the post-surgical visual performance of the plurality of intraocular lenses (2i) in the eye (1) of the patient are computed by performing for each intraocular lens (2i) of the plurality of intraocular lenses (2i) the following steps:
estimating a post-surgical position of the intraocular lens (2i) in the pre-surgical eye model based on the pre-surgical eye measurement (ME) and based on lens data of the intraocular lens;
generating a post-surgical optical model (OEi) of the eye (1) by combining the pre-surgical optical model of the eye (1) with an optical lens model (OLi) of the intraocular lens (2i) at the estimated post-surgical position in the pre-surgical eye model;
simulating the post-surgical visual performance of the intraocular lens (2i) based on the post-surgical optical model (OEi) of the eye (1);
**characterized in**
a lens measurement equipment (3) for measuring the plurality of intraocular lenses (2i) to obtain a plurality of lens measurements (MLi) of the intraocular lenses (2i), wherein the processing means (5) is configured for obtaining the optical lens model (OLi) of each intraocular lens (2i) measured by the lens measurement equipment (3) based on its lens measurement (MLi).

15. Computer program for selecting an intraocular lens (2i) to be implanted into an eye (1) of a patient, the computer program comprising a set of instruction which, when executed on a processing means (5), perform the following steps:
storing for each of a plurality of intraocular lenses (2i) an optical lens model (OLi) and lens data;
receiving a pre-surgical eye measurement (ME) of the eye (1) of the patient;
generating a pre-surgical optical model of the eye (1) of the patient based on the pre-surgical eye measurement (ME);
computing the post-surgical visual performance of the plurality of intraocular lenses (2i) in the eye (1) of the patient by performing for each intraocular lens (2i) of the plurality of intraocular lenses (2i) the following steps:
estimating a post-surgical position of the intraocular lens (2i) in the pre-surgical eye model based on the pre-surgical eye measurement (ME) and based on the lens data of the intraocular lens;
generating a post-surgical optical model (OEi) of the eye (1) by combining the pre-surgical optical model of the eye (1) with the optical lens model (OLi) at the estimated post-surgical position in the pre-surgical eye model; and
simulating the post-surgical visual performance of the intraocular lens (2i) based on the post-surgical optical model (OEi) of the eye (1); and
outputting a selection advise for selecting the intraocular lens (2i) to be implanted in the eye (1) of the patient based on the computed post-surgical visual performances of the plurality of intraocular lenses (2i);
**characterized in that** the stored optical lens models (OLi) of the intraocular lenses (2i) are obtaining based on lens measurements (MLi) of the respective intraocular lenses (2i).
